# EUROPÄISCHE PATENTSCHRIFT

(11) **EP 3 652 137 B1**
(45) Veröffentlichungstag und Bekanntmachung des Hinweises auf die Patenterteilung: **01.09.2021**
(21) Anmeldenummer: 18737307.1
(22) Anmeldetag: 12.07.2018
(51) Int. Cl.: C07C 5/333, C07C 7/00, C07C 7/04, C07C 7/09, C07C 9/08, C07C 11/06, C10G 9/36

(54) **PROZESS UND ANLAGE ZUR HERSTELLUNG VON PROPYLEN DURCH KOMBINATION VON PROPANDEHYDRIERUNG UND DAMPFSPALTVERFAHREN MIT PROPAN-RÜCKFÜHRUNG IN DAS DAMPFSPALTVERFAHREN**
PROCESS AND PLANT FOR PRODUCING PROPYLENE BY COMBINATION OF PROPANE DEHYDRATION AND STEAM CRACKING WITH RECYCLING OF PROPANE INTO THE STEAM CRACKING PROCESS
PROCÉDÉ ET INSTALLATION DE FABRICATION DE PROPYLÈNE PAR COMBINAISON DE DÉSHYDROGÉNATION DE PROPANE ET PROCÉDÉ DE VAPOCRAQUAGE AVEC RECYCLAGE DE PROPANE AU PROCÉDÉ DE VAPOCRAQUAGE

(30) Priorität: 12.07.2017 EP 17180984
(43) Veröffentlichungstag der Anmeldung: 20.05.2020
(73) Patentinhaber: Linde GmbH, 82049 Pullach (DE)
(72) Erfinder: HÖFEL, Torben, 81543 München (DE); TÖGEL, Christine, 80331 München (DE); ZELLHUBER, Mathieu, 82152 Planegg (DE); LAIB, Heinrich, 67056 Ludwigshafen (DE); KOTREL, Stefan, 67056 Ludwigshafen (DE); DIETERLE, Martin, 67056 Ludwigshafen (DE); PATCAS, Florina Corina, 67056 Ludwigshafen (DE); GIESA, Sonja, 67056 Ludwigshafen (DE)
(86) Internationale Anmeldenummer: PCT/EP2018/068967
(87) Internationale Veröffentlichungsnummer: WO 2019/012051

(56) Entgegenhaltungen:
- WO-A1-2015/128039
- CN-A- 103 086 826
- DE-A1-102005 053 732

## Beschreibung

Die Erfindung betrifft einen Prozess und eine Anlage zur Herstellung von Propylen sowie ein Verfahren zum Umrüsten einer Anlage zum Dampfspalten gemäß den Oberbegriffen der unabhängigen Patentansprüche.

### Stand der Technik

Propylen (Propen) wird herkömmlicherweise überwiegend durch Dampfspalten (engl. Steam Cracking) von Kohlenwasserstoffeinsätzen und weitere Umwandlungsverfahren im Zuge von Raffinerieprozessen hergestellt. In diesen Fällen stellt Propylen ein in geringerem Umfang anfallendes Nebenprodukt dar. Aufgrund des zunehmenden Bedarfs an Propylen, insbesondere für Polypropylen, wird daher auch die Propandehydrierung eingesetzt.

Die Propandehydrierung ist ein allgemein bekanntes Verfahren der petrochemischen Industrie und beispielsweise im Artikel "Propene" in Ullmann's Encyclopedia of Industrial Chemistry, Onlineausgabe 16. September 2013, DOI: 10.1002/14356007.a22_211 .pub3, insbesondere Abschnitt 3.3.1, "Propane Dehydrogenation" beschrieben.

Bei der Propandehydrierung handelt es sich um eine endotherme Gleichgewichtsreaktion, die im Allgemeinen an Edel- oder Schwermetallkatalysatoren, umfassend beispielsweise Platin oder Chrom, durchgeführt wird. Die Dehydrierungseaktion ist ausgesprochen selektiv. Für kommerziell verfügbare Prozesse werden Gesamtausbeuten von ca. 90% genannt. Ungeachtet dieser hohen Selektivität entstehen neben dem abgespaltenen Wasserstoff jedoch typischerweise auch geringere Mengen an Kohlenwasserstoffen mit einem, zwei sowie vier und mehr als vier Kohlenstoffatomen als Nebenprodukte. Diese Nebenprodukte müssen vom Zielprodukt Propylen abgetrennt werden.

Dampfspaltverfahren und Raffinerieprozesse, in denen Propylen gebildet wird, sind ebenfalls in der Literatur beschrieben, beispielsweise im Artikel "Ethylene" in Ullmann's Encyclopedia of Industrial Chemistry, Onlineveröffentlichung 15. April 2009, DOI: 10.1002/14356007.a10_045.pub3, und im Artikel "Oil Refining" in Ullmann's Encyclopedia of Industrial Chemistry, Onlineveröffentlichung 15. Januar 2007, DOI: 10.1002/14356007.a18_051.pub2.

Grundsätzlich kann eine Aufreinigung eines bei der Propandehydrierung erzeugten Komponentengemischs zumindest teilweise zusammen mit der Aufreinigung eines Propylen enthaltenden Komponentengemischs aus einem anderen Verfahren, in dem Propylen gebildet wird, beispielsweise einem Dampfspaltverfahren oder einem Raffinerieprozess, erfolgen.

Die Kombination der Aufreinigung eines Komponentengemischs aus einer Propandehydrierung mit der Aufreinigung eines Komponentengemisch aus einem Dampfspaltverfahren ist beispielsweise aus der US 4,458,096 A oder, spezifisch bezogen auf eine Spaltung von Kohlenwasserstoffen mit zwei Kohlenstoffatomen, aus der WO 2015/128039 A1 bekannt, eine entsprechende Kombination mit der Aufreinigung eines Komponentengemisches aus einem Fluid-Catalytic-Cracking-Verfahren beispielsweise aus der US 8,563,793 A oder der US 2010/331589 A1. Genauere Ausführungen bezüglich einer entsprechenden Kombination enthalten diese Druckschriften jedoch nicht. Ferner sei bereits an dieser Stelle darauf hingewiesen, dass ein Fluid-Catalytic-Cracking-Verfahren Produktgemische mit grundsätzlich anderer Zusammensetzung als ein Dampfspaltverfahren liefert, so dass eine kombinierte Trennung hier abweichend ausgestaltet werden muss.

In der bereits erwähnten WO 2015/128039 A1 ist beschrieben, dass ein Komponentengemisch mit überwiegend oder ausschließlich zwei Kohlenstoffatomen, welches in einer gemeinsamen Trennung gebildet wird, der ein Komponentengemisch, das unter Einsatz des Verfahrens zur Propandehydrierung gebildet wird, und ein Komponentengemisch, das unter Einsatz des Dampfspaltverfahrens gebildet wird, unterworfen werden, zu dem Dampfspaltverfahren zurückgeführt wird. Ein in der gemeinsamen Trennung gebildetes Komponentengemisch mit drei und mehr Kohlenstoffatomen wird hingegen, da es beträchtliche Mengen an Propan aufweist, zu dem Verfahren zur Propandehydrierung zurückgeführt. Auch andere Druckschriften offenbaren eine Rückführung von Propan bzw. entsprechender Propan enthaltender Komponentengemische zu einem Verfahren zur Propandehydrierung.

Aus der CN 103 086 826 A ist ein Verfahren zur Herstellung von Ethylen und Propylen bekannt. Es umfasst ein Dampfspalten von Ethan und eine katalytische Dehydrierung von Propan. Ein weiteres vorgeschlagenes Verfahren umfasst die Verwendung eines Ethylenspaltsystems, welches eine katalytische Dehydrierungsvorrichtung aufweist, die im Konvektionsabschnitt eines Ethanspaltofens angeordnet ist. Die Verfahren sollen die Vorteile einer effektiven Reduzierung des Energieverbrauchs eines voreingestellten Dampfspaltsystems, der gleichzeitigen Implementierung des Ethan-Dampfspaltens und der katalytischen Propandehydrierung durch den Einsatz des Ethanspaltofens, sowie der Energie- und Kostenreduzierung haben.

Die DE 10 2005 053732 A1 offenbart ein Verfahren zur Erzeugung von Propylen aus einem ersten, Alkane enthaltenden, jedoch propanarmen Stoffstrom und einem zweiten, propanreichen Stoffstrom, umfassend die Schritte des Erzeugens eines ersten Zwischenproduktstromes durch Dampfspalten des ersten Stoffstromes, des Erzeugens eines zweiten Zwischenproduktstromes durch katalytisches Dehydrieren zweiten Stoffstromes, des Zusammenführen der beiden Zwischenproduktströme zu einem dritten Zwischenproduktstrom und des Zerlegens des dritten Zwischenproduktstromes, wobei zumindest Ethylen und Propylen als Endprodukte gewonnen werden.

Die vorliegende Erfindung stellt sich die Aufgabe, Prozesse zur Herstellung von Propylen, bei denen ein Komponentengemisch aus einem Verfahren zur Propandehydrierung aufgereinigt wird, und bei dem bestimmte Komponenten bzw. Fraktionen zurückgeführt werden, zu verbessern und effizienter zu gestalten, wenn ein zusätzliches Dampfspaltverfahren zur Verfügung steht.

### Offenbarung der Erfindung

Vor diesem Hintergrund schlägt die vorliegende Erfindung einen Prozess und eine Anlage zur Herstellung von Propylen sowie ein Verfahren zum Umrüsten einer Anlage zur Durchführung eines Dampfspaltverfahrens mit den jeweiligen Merkmalen der unabhängigen Patentansprüche vor. Bevorzugte Ausgestaltungen sind Gegenstand der abhängigen Patentansprüche sowie der nachfolgenden Beschreibung.

Im Rahmen der vorliegenden Erfindung wurde erkannt, dass paradoxerweise eine Rückführung eines überwiegend Propan enthaltenden Trennungsprodukts nicht in das Verfahren zur Propandehydrierung sondern in das andere Propanherstellungsverfahren vorteilhaft ist, wenn das überwiegend Propan enthaltende Trennungsprodukt in einer Trennung gebildet wird, welcher zumindest ein Teil eines ersten Komponentengemischs, das unter Verwendung eines Verfahrens zur Propandehydrierung bereitgestellt wird, und optional auch zumindest ein Teil eines zweiten Komponentengemischs, das unter Verwendung eines weiteren Propanherstellungsverfahrens bereitgestellt wird, unterworfen werden. Dies gilt insbesondere dann, wenn es sich bei dem anderen Propanherstellungsverfahren um ein Dampfspaltverfahren handelt, welches daher erfindungsgemäß eingesetzt wird.

Diese Rückführung ist kontraintuitiv, da das überwiegend Propan enthaltende Trennungsprodukt vermeintlich einen besonders wertvollen Einsatz für das Verfahren zur Propandehydrierung darstellt, da ja in der Propandehydrierung Propan zu Propylen umgesetzt werden soll und daher bekanntermaßen ein propanreicher Einsatz verwendet wird. Daher wäre es, falls eine Rückführung erfolgt, ausgesprochen naheliegend, ein Propan enthaltendes Komponentengemisch in das Verfahren zur Propandehydrierung zurückzuführen, wie dies auch die WO 2015/128039 A1 in Bezug auf ein Komponentengemisch vorschlägt, das Propan und schwerere Kohlenwasserstoffe aufweist. Die vorliegende Erfindung hat gerade das Gegenteil als vorteilhaft erkannt.

Propanhaltige Einsätze für die Propandehydrierung liegen typischerweise mit hoher Propankonzentration vor (z.B. mindestens 94 Volumenprozent Propan und jeweils höchstens 4 Volumenprozent Butan, 3 Volumenprozent Ethan, und 0,1 Volumenprozent Olefine). Verglichen damit hat ein überwiegend Propan enthaltendes Trennungsprodukt aus einer Trennung, insbesondere dann, wenn dieser gemeinsamen Trennung auch ein Komponentengemisch unterworfen wird, das unter Verwendung eines Dampfspaltverfahrens bereitgestellt wird, gegebenenfalls eine mindere Qualität. Dies gilt zumindest dann, wenn in der Trennung kein unverhältnismäßig großer Aufwand bezüglich der Reinheit der gewonnenen Produkte betrieben wird. In einem entsprechenden, überwiegend Propan enthaltenden Trennungsprodukt liegen daher Olefine, insbesondere einfach und mehrfach ungesättigte Olefine mit drei Kohlenstoffatomen und einfach und mehrfach ungesättigte Olefine mit vier Kohlenstoffatomen, vor.

Je nach Gestaltung des Verfahrens zur Propandehydrierung weist ein verwendeter Reaktor bzw. dessen Katalysator gegebenenfalls ausgesprochen geringe Toleranzen hinsichtlich Komponenten wie Methylacetylen, Propadien und Butadien auf, die regelmäßig in entsprechenden anderen Propylenherstellungsverfahren, insbesondere in Dampfspaltverfahren, gebildet werden. Eine Rückführung zu dem Verfahren zur Propandehydrierung würde daher erfordern, die genannten Komponenten zumindest weitgehend abzutrennen, um die Toleranzen eines entsprechenden Reaktors einzuhalten. Dies wäre jedoch mit einem unverhältnismäßig hohen apparativen und verfahrenstechnischen Aufwand verbunden.

Ein Prozess gemäß einer besonders bevorzugten Ausführungsform der Erfindung umfasst eine kombinierte Aufreinigung bzw. Trennung von Komponentengemischen aus dem Verfahren zur Propandehydrierung und dem Dampfspaltverfahren. Der erfindungsgemäße Prozess kann jedoch grundsätzlich auch ohne eine kombinierte Aufreinigung bzw. Trennung durchgeführt werden. Für eine kombinierte Aufreinigung von Komponenten aus unterschiedlichen Verfahren ist es besonders vorteilhaft, wenn die jeweiligen Komponentengemische gleiche bzw. ähnliche Komponenten enthalten, d.h. die Komponentengemische sich nicht gegenseitig mit bestimmten, in dem jeweils anderen Komponentengemisch nicht enthaltenen Komponenten "kontaminieren" (beispielsweise mit Wasserstoff, Kohlendioxid oder Oxygenaten).

Eine Kombination der Aufreinigung ist insbesondere dann vorteilhaft, wenn die jeweiligen Komponentengemische einen ähnlichen Konzentrationsbereich aufweisen, so dass ein synergetischer Trennprozess erwartet werden kann. Dies ist jedoch in der Praxis normalerweise nicht der Fall. Ferner erfolgt eine Kombination der Aufreinigung vorteilhafterweise dann, wenn eines der Verfahren deutlich geringere Mengen eines entsprechenden Komponentengemischs liefert bzw. eine entsprechende Anlage kleiner ausgebildet und daher eine separate Aufreinigung nicht lohnenswert ist. Dies kann insbesondere der Fall sein, wenn das andere Propylenherstellungsverfahren, beispielsweise ein Dampfspaltverfahren, bereits in Form einer Anlage realisiert ist und eine Propandehydrierung zur Erhöhung der Propylenproduktkapazität mit deutlich geringer Kapazität nachgerüstet wird. Dies könnte insbesondere sinnvoll vorteilhaft sein, wenn einige Anlagenteile zur Durchführung des Dampfspaltverfahrens aufgrund einer späteren Einsatzänderung nicht mehr mit voller Kapazität laufen und diese Kapazitäten von dem Propandehydrierungsverfahren genutzt werden können.

Sofern das Dampfspaltverfahren bereits zur Verwendung eines propanhaltigen Einsatzes ausgelegt ist, kann dieses auch nach der Nachrüstung mit Propan weiterbetrieben werden. Jedoch erhält das Dampfspaltverfahren nach der Nachrüstung zumindest teilweise das überwiegend Propan enthaltende Trennungsprodukt, was keine großen Auswirkungen auf das Verfahren hat. Das Verfahren zur Propandehydrierung erhält den Propaneinsatzstoff mit höherer Qualität aus einer anderen Quelle (und ggf. auch zumindest einen Teil des überwiegend Propan enthaltenden Trennungsproduktes). Entsprechend muss der (gemeinsame) Trennteil hinsichtlich des überwiegend Propan enthaltenden Trennungsproduktes nicht auf die Anforderungen des Verfahrens zur Propandehydrierung ausgelegt werden, d.h. insbesondere sind keine Schritte zur (separaten) Hydrierung und keine Schritte zur Abtrennung von Kohlenwasserstoffen mit vier und mehr Kohlenstoffatomen erforderlich, um die Toleranzen des Verfahrens zur Propandehydrierung einzuhalten.

Eine Möglichkeit zur vorteilhaften Ausgestaltung der bevorzugten Ausführungsform der vorliegenden Erfindung, bei der eine kombinierte Aufreinigung bzw. Trennung zum Einsatz kommt, ist es daher, ein erstes Komponentengemisch, das unter Verwendung des Verfahrens zur Propandehydrierung erhalten wird, derart vorzubehandeln, dass dieses in einem an (zumindest) Wasserstoff abgereicherten Zustand und insbesondere bei einem erhöhten Druck vorliegt. Hierbei wird das erste Komponentengemisch einem oder mehreren, Vortrennschritten unterworfen, die nachfolgend auch als "erste" Vortrennschritte bezeichnet werden. Das auf diese Weise vorbehandelte Komponentengemisch, das nachfolgend, wenn konkret hierauf Bezug genommen wird, auch als "drittes" Komponentengemisch bezeichnet wird, enthält aufgrund seiner Vorbehandlung überwiegend Kohlenwasserstoffe mit drei Kohlenstoffatomen. Ferner können geringere Mengen an Methan, Restwasserstoff sowie Kohlenwasserstoffe mit zwei Kohlenstoffatomen sowie Kohlenwasserstoffe mit vier und gegebenenfalls mehr als vier Kohlenstoffatomen enthalten sein.

In der soeben erläuterten Ausgestaltung der bevorzugten Ausführungsform der vorliegenden Erfindung ist es ferner vorgesehen, ein zweites Komponentengemisch, das unter Verwendung des Dampfspaltverfahrens erhalten wird, derart vorzubehandeln, dass dieses in einem (zumindest) an Wasserstoff und Methan abgereicherten Zustand und insbesondere ebenfalls bei einem erhöhten Druck vorliegt. Hierbei wird das zweite Komponentengemisch einem oder mehreren Vortrennschritten unterworfen, die nachfolgend auch als "zweite Vortrennschritte" bezeichnet werden. Das auf diese Weise vorbehandelte Komponentengemisch, das nachfolgend, wenn konkret hierauf Bezug genommen wird, auch als "viertes" Komponentengemisch bezeichnet wird, enthält aufgrund seiner Vorbehandlung vorteilhafterweise überwiegend ähnliche Kohlenwasserstoffe, wie sie in dem dritten Komponentengemisch enthalten sind, und diese in einem vergleichbaren Konzentrationsbereich, sowie vergleichbare Mengen an Restwasserstoff und Restmethan, soweit nicht vollständig abgetrennt.

Da grundsätzlich bei einem alternativ oder zusätzlich zu einem Verfahren zur Propandehydrierung eingesetzten Propylenherstellungsverfahren, insbesondere einem Dampfspaltverfahren, größere Mengen an Kohlenwasserstoffen mit zwei Kohlenstoffatomen, insbesondere Ethan und Ethylen, gebildet werden können, insbesondere bei Einsatz von leichteren Dampfspalteinsätzen, kann im Zuge des oder der zweiten Vortrennschritte auch eine Abreicherung an Kohlenwasserstoffen mit zwei Kohlenstoffatomen im Zuge der Abreicherung an Wasserstoff und Methan erfolgen. Mit anderen Worten kann im Zuge des oder der zweiten Vortrennschritte ein Demethanizer-First-Verfahren oder ein Deethanizer-First-Verfahren zum Einsatz kommen. Auch der Einsatz eines Depropanizer-First-Verfahrens ist grundsätzlich möglich. Weitere Details werden nachfolgend erläutert. Auch im Zuge des oder der ersten Vortrennschritte kann jedoch bereits eine Abreicherung an Kohlenwasserstoffen mit zwei Kohlenstoffatomen erfolgen, falls dies zweckmäßig ist. Weitere Aspekte dieser Ausführungsformen werden unten erläutert.

Die durch den oder die ersten Vortrennschritte und durch den oder die zweiten Vortrennschritte in ihrer Zusammensetzung und ihrem Druck zumindest teilweise aneinander angeglichenen dritten und vierten Komponentengemische können in besonders vorteilhafter Weise vereinigt und anschließend nachfolgenden, gemeinsamen Trennschritten unterworfen werden. Dies ermöglicht es, entsprechende Anlagenteile für beide Verfahren gemeinsam auszubilden und damit eine entsprechende Anlage mit geringeren Investitionskosten zu erstellen und/oder mit geringeren Betriebskosten zu betreiben.

Ist hier davon die Rede, dass ein Komponentengemisch gegenüber einem anderen Komponentengemisch, hier insbesondere das dritte gegenüber dem ersten und das vierte gegenüber dem zweiten, an einer oder an mehreren Komponenten, hier insbesondere an Wasserstoff bzw. Wasserstoff und Methan, "abgereichert" ist, sei hierunter verstanden, dass das abgereicherte Komponentengemisch höchstens den 0,5-fachen, 0,2-fachen, 0,1-fachen, 0,01-fachen oder 0,001-fachen Gehalt an der einen oder den mehreren Komponenten, bezogen auf das nicht abgereicherte Komponentengemisch und auf molarer, Masse- oder Volumenbasis, enthält. Auch eine vollständige Entfernung, d.h. eine "Abreicherung auf null" wird hier als Abreicherung verstanden. Nachfolgend bezeichnet der Begriff "überwiegend" einem Gehalt von wenigstens 60%, 80%, 90%, 95% oder 99% auf molarer, Masse- oder Volumenbasis.

Insgesamt schlägt die vorliegende Erfindung dabei einen Prozess zur Herstellung von Propylen vor, der das Durchführen eines Verfahrens zur Propandehydrierung unter Erhalt eines ersten Komponentengemischs, das zumindest Wasserstoff, Ethan, Ethylen, Propan und Propylen enthält, und das Durchführen eines Dampfspaltverfahrens, unter Erhalt eines zweiten Komponentengemischs, das zumindest Wasserstoff, Methan, Ethan, Ethylen, Propan und Propylen enthält, vor. Zu den Details der jeweiligen Verfahren und den dabei typischerweise gebildeten Produktzusammensetzungen, insbesondere auch bezüglich zusätzlich zu den erwähnten Komponenten enthaltener Verbindungen, sei auf die mehrfach zitierte Fachliteratur verwiesen. Dem Verfahren zur Propandehydrierung werden dabei vorteilhafterweise Propan enthaltende Einsätze, dem Dampfspaltverfahren kohlenwasserstoffreiche Einsätze zugeführt. Bei letzteren handelt es sich beispielsweise um Naphtha, aber gegebenenfalls auch leichtere oder schwerere Einsätze, also solche, die leichter und/oder schwerer siedende Kohlenwasserstoffe enthalten, als sie typischerweise in Naphtha vorhanden sind.

Wie insoweit auch in kombinierten Prozessen üblich, erfolgt dabei ein Bilden eines überwiegend Propan enthaltenden Trennungsprodukts unter Verwendung zumindest eines Teils des Propans des ersten Komponentengemischs und unter Einsatz eines oder mehrerer Propanabtrennschritte. Gemäß der zuvor erläuterten bevorzugten Ausführungsform der vorliegenden Erfindung kann das überwiegend Propan enthaltende Trennungsprodukt auch unter Verwendung zumindest eines Teils des Propans des zweiten Komponentengemischs, also mittels einer gemeinsamen Aufreinigung oder Trennung, gebildet werden. Der oder die Propanabtrennschritte können dabei insbesondere die Verwendung eines sogenannten C3-Splitters umfassen, dem kopfseitig ein überwiegend oder ausschließlich Propylen enthaltendes Trennungsprodukt und dem sumpfseitig das überwiegend Propan enthaltende Trennungsprodukt entnommen werden kann. Einem derartigen C3-Splitter sind typischerweise weitere Trennschritte vorgeschaltet, wie sie auch nachfolgend noch erläutert werden.

Insbesondere kann der Einsatz eines entsprechenden C3-Splitters typischerweise einem sogenannten Depropanizer, bzw. einer entsprechenden Depropanizerkolonne, entnommen werden wie grundsätzlich ebenfalls aus der zitierten Fachliteratur bekannt. Bei einem Depropanizer handelt es sich um eine Rektifikationskolonne, der kopfseitig eine gasförmige Fraktion, die überwiegend oder ausschließlich Kohlenwasserstoffe mit drei Kohlenstoffatomen enthält, entnommen werden kann, und der sumpfseitig eine flüssige Fraktion, die überwiegend oder ausschließlich Kohlenwasserstoffe mit vier und gegebenenfalls mehr Kohlenstoffatomen enthält, entnommen werden kann. Auch weiteren, einer entsprechenden Rektifikationskolonne zugeordneten Apparaten, die Teil eines entsprechenden Depropanizers sind, beispielsweise Absorbern, können anstelle der Rektifikationskolonne selbst oder zusätzlich zu dieser, entsprechende Fraktionen entnommen werden. Die kopfseitig dem Depropanizer oder entsprechenden Apparaten entnommene Fraktion kann dem C3-Splitter zugeführt werden. Ein entsprechender Depropanizer kann an unterschiedlicher Stelle in einer Trennsequenz zur Bearbeitung eines Komponentengemischs, insbesondere eines durch ein Dampfspaltverfahren oder ein anderes Propanherstellungsverfahren erhaltenen Komponentengemischs, angeordnet sein. Insbesondere kann ein entsprechender Depropanizer im Rahmen der vorliegenden Erfindung stromab eines Deethanizers bzw. einer entsprechenden Deethanizerkolonne angeordnet und zur trenntechnischen Bearbeitung eines Sumpfprodukts des Deethanizers eingerichtet sein. Wie auch nachfolgend erläutert, kann im Rahmen der vorliegenden Erfindung ein Deethanizer im Zuge des oder der zweiten Vortrennschritte oder bereits im Zuge des oder der Propanabtrennschritte eingesetzt werden, abhängig davon, ob ein Deethanizer-First-Verfahren oder ein Demethanizer-First-Verfahren zum Einsatz kommt. Entsprechendes gilt auch für einen Depropanizer, der ebenfalls an erster Stelle einer entsprechenden Trennsequenz stehen kann.

Dem oder den im Rahmen der vorliegenden Erfindung eingesetzten Propanabtrennschritten wird stets zumindest ein Teil des ersten Komponentengemischs und, gemäß der Ausführungsform mit gemeinsamer Aufreinigung bzw. Trennung, auch zumindest ein Teil des zweiten Komponentengemischs zugeführt. Unter "zumindest einem Teil" sei dabei auch verstanden, dass dem oder den Propanabtrennschritten eine, beispielsweise in dem oder den zuvor erläuterten Vortrennschritten gebildete, Fraktion zugeführt wird. Mit anderen Worten muss es sich also bei einem "Teil" nicht um eine Teilmenge mit identischer Zusammensetzung handeln, vielmehr kann ein "Teil" auch eine Fraktion mit abweichender Zusammensetzung sein, insbesondere ein weiteres Komponentengemisch, das im Rahmen eines bereits erwähnten (ersten oder zweiten) Vortrennschritts gebildet wird. Mit anderen Worten kann dem oder den im Rahmen der vorliegenden Erfindung eingesetzten Propanabtrennschritten also zumindest ein Teil (im Sinne einer Teilmenge) des ersten Komponentengemischs und optional zumindest ein Teil (im Sinne einer Teilmenge) des zweiten Komponentengemischs zugeführt werden, es ist jedoch auch möglich, dem oder den Propanabtrennschritten ein oder mehrere Komponentengemische (nämlich insbesondere die bereits erwähnten "dritten" und "vierten" Komponentengemische) zuzuführen, die unter Verwendung zumindest eines Teils des ersten Komponentengemischs und zumindest eines Teils des zweiten Komponentengemischs gebildet werden.

Die Vorteile des wesentlichen Aspekt der vorliegenden Erfindung, gemäß dem das überwiegend Propan enthaltende Trennungsprodukt zumindest zum Teil in das Dampfspaltverfahren zurückgeführt wird, wurden bereits zuvor erläutert. Diese bestehen, wie erwähnt, insbesondere darin, dass auf diese Weise vermieden werden kann, dass dem Verfahren zur Propandehydrierung mit diesem nicht kompatible Komponenten, insbesondere ein- und mehrfach ungesättigte Kohlenwasserstoffe mit drei und mehr Kohlenstoffatomen zugeführt werden. Wie ebenfalls erwähnt, kann auf diese Weise darauf verzichtet werden, dass überwiegend Propan enthaltende Trennungsprodukt einer aufwändigen Reinigung zu unterwerfen, da in dem Dampfspaltverfahren, zumindest Restgehalte entsprechender Komponenten toleriert und gegebenenfalls sogar umgesetzt werden können. Auf diese Weise kann der erfindungsgemäß vorgeschlagene Prozess im Gegensatz zu anderen Prozessen, die Verfahren zur Propandehydrierung entsprechende rückgeführte Ströme zuführen, einfacher und kostengünstiger durchgeführt werden. Für das Verfahren zur Propandehydrierung kann im Rahmen der vorliegenden Erfindung stets auf Frischeinsätze mit entsprechender Spezifikation zurückgegriffen werden.

Ist hier davon die Rede, dass das überwiegend Propan enthaltende Trennungsprodukt "zumindest zum Teil" in das Dampfspaltverfahren zurückgeführt wird, fällt hierunter auch eine Konstellation, gemäß derer ein Teil des überwiegend Propan enthaltenden Trennungsprodukts in das Dampfspaltverfahren und ein weiterer Teil des überwiegend Propan enthaltenden Trennungsprodukts in das Verfahren zur Propandehydrierung zurückgeführt wird. Auf diese Weise kann in dem Verfahren zur Propandehydrierung beispielsweise durch eine entsprechende "Verdünnung" mit Frischeinsatz der Gehalt an störenden Komponenten derart reduziert werden, dass diese in dem Verfahren nicht mehr stören. Mit anderen Worten kann hier eine Zudosierung des überwiegend Propan enthaltenden Trennungsprodukts erfolgen, die stets nur in dem Umfang erfolgt, dass sie das Verfahren zur Propandehydrierung nicht stört. Der Rest des überwiegend Propan enthaltenden Trennungsprodukts kann hingegen in das Dampfspaltverfahren zurückgeführt werden. Gemäß einer besonders bevorzugten Ausgestaltung der vorliegenden Erfindung wird jedoch das überwiegend Propan enthaltende Trennungsprodukt nicht in das Verfahren zur Propandehydrierung zurückgeführt, so dass dem Verfahren zur Propandehydrierung in dieser Ausgestaltung des erfindungsgemäßen Verfahrens lediglich Propan zugeführt wird, das weder aus dem ersten noch aus dem zweiten Komponentengemisch stammt.

Wie erwähnt kann im Rahmen der vorliegenden Erfindung durch die Rückführung des überwiegend Propan enthaltenden Trennungsprodukts auf eine aufwendige Reinigung verzichtet werden. Mit anderen Worten kann ein nur überwiegend Propan enthaltendes Trennungsprodukt verwendet werden, das in einem Gehalt von 0,1 bis 25 Volumenprozent einfach und mehrfach ungesättigte Kohlenwasserstoffe, insbesondere einfach und mehrfach ungesättigte Kohlenwasserstoffe mit drei und vier Kohlenstoffatomen, insbesondere Propadien und Butadien, enthalten kann. Ein derartiges Trennungsprodukt kann in einem weiteren Propylenherstellungsverfahren, insbesondere einem Dampfspaltverfahren, in höheren Grenzen als im Verfahren zur Propandehydrierung toleriert werden.

Wie bereits erwähnt, werden im Rahmen eines entsprechenden Prozesses typischerweise weitere Produkte gebildet. So umfasst ein Prozess gemäß einer besonders bevorzugten Ausführungsformen der vorliegenden Erfindung ferner das Bilden eines überwiegend oder ausschließlich Propylen enthaltenden Trennungsprodukts unter Verwendung zumindest eines Teils des Problems des ersten und des zweiten Komponentengemischs und unter Einsatz des oder der Propanabtrennschritte. Wie bereits erläutert, kann in diesem Zusammenhang ein C3-Splitter zum Einsatz kommen.

Die vorliegende Erfindung umfasst in der soeben erläuterten Ausgestaltung ferner das Bilden eines Trennungsprodukts, das überwiegend oder ausschließlich Ethylen enthält, unter Verwendung zumindest eines Teils des Ethylens des ersten und des zweiten Komponentengemischs und unter Einsatz eines oder mehrerer weiterer Trennschritte, sowie das Bilden eines Trennungsprodukts, das oder ausschließlich überwiegend Ethan enthält, unter Verwendung zumindest eines Teils des Ethans des ersten und des zweiten Komponentengemischs und unter Einsatz des oder der weiteren Trennschritte. Der oder die weiteren Trennschritte umfassen dabei typischerweise die Verwendung eines sogenannten C2-Splitters, dem kopfseitig das überwiegend oder ausschließlich Ethylen enthaltende Trennungsprodukt und sumpfseitig das überwiegend oder ausschließlich Ethan enthaltende Trennungsprodukt entnommen werden kann. Ein entsprechender C2-Splitter kann insbesondere mit einer überwiegend oder ausschließlich Ethan und Ethylen enthaltenen Fraktion gespeist werden, die in einem Demethanizer-First-Verfahren vom Kopf eines Deethanizers, also einer entsprechenden Rektifikationskolonne oder einem dieser zugeordneten Apparat, und in einem Deethanizer-First-Verfahren aus dem Sumpf eines Demethanizers abgezogen werden kann.

Wie erwähnt, umfasst die vorliegende Erfindung gemäß besonders bevorzugter Ausführungsformen Vortrennschritte, wobei vorgesehen ist, dass zumindest ein Teil des ersten Komponentengemischs unter Erhalt eines dritten Komponentengemischs einem oder mehreren ersten Vortrennschritten unterworfen wird, der oder die eine Druckerhöhung und eine zumindest teilweise Entfernung von Wasserstoff umfassen, und dass zumindest ein Teil des zweiten Komponentengemischs unter Erhalt eines vierten Komponentengemischs einem oder mehreren zweiten Vortrennschritten unterworfen wird, der oder die eine Druckerhöhung, eine zumindest teilweise Entfernung von Wasserstoff und eine zumindest teilweise Entfernung von Methan umfassen. Bereits an dieser Stelle sei darauf hingewiesen, dass, wie bereits oben erwähnt, im Rahmen des oder der zweiten Vortrennschritte auch eine zumindest teilweise Entfernung von Kohlenwasserstoffen mit zwei Kohlenstoffatomen vorgenommen werden kann. In letzterem Fall kommt die vorliegende Erfindung im Zusammenhang mit einem Deethanizer-First-Verfahren zum Einsatz, ansonsten im Zusammenhang mit einem Demethanizer-First-Verfahren. Wie erwähnt, kann die vorliegende Erfindung auch im Zusammenhang mit einem Depropanizer-First-Verfahren eingesetzt werden.

Ferner ist im Rahmen der vorliegenden Erfindung gemäß der soeben erläuterten Ausführungsform in einer Alternative vorgesehen, dass zumindest ein Teil des dritten Komponentengemischs gemeinsam mit zumindest einem Teil des vierten Komponentengemischs dem oder den Propanabtrennschritten unterworfen wird. Gemäß einer weiteren Alternative ist vorgesehen, dass zumindest ein Teil des dritten Komponentengemischs zusammen mit dem zweiten Komponentengemisch unter Bildung des vierten Komponentengemischs dem oder den zweiten Vortrennschritten unterworfen wird und das vierte Komponentengemisch dem oder den Propanabtrennschritten unterworfen wird. Wie bereits erwähnt, können also das dritte und das vierte Komponentengemisch im oben erläuterten Sinn "Teile" des ersten bzw. des zweiten Komponentengemischs sein. Die vorliegende Erfindung sieht dabei in ihren nachfolgend erläuterten, jeweiligen Ausgestaltungen unterschiedliche Möglichkeiten zur Vereinigung des dritten Komponentengemischs mit dem vierten Komponentengemisch bzw. ihrer jeweils verwendeten Anteile vor. In sämtlichen Fällen besteht ein Hauptvorteil der vorliegenden Erfindung darin, dass aufgrund einer vergleichbaren Zusammensetzung des dritten und vierten Komponentengemischs eine besonders einfache und effiziente gemeinsame Trennung möglich ist.

Wie bereits erwähnt, kann die vorliegende Erfindung im Zusammenhang mit einem Deethanizer-First-Verfahren zum Einsatz kommen. In diesem Fall werden bei der Entfernung von Wasserstoff und Methan in dem oder den zweiten Vortrennschritten auch Ethan und Ethylen zumindest überwiegend entfernt, so dass diese überwiegend nicht aus dem zweiten in das vierte Komponentengemisch bzw. entsprechende Anteile übergehen. Es sei darauf hingewiesen, dass in einem entsprechenden Deethanizer-First-Verfahren das vierte Komponentengemisch auch eine Flüssigkeit sein kann, die auf einem sumpfnahen Trennboden einer entsprechenden Rektifikationskolonne vorliegt. Unter einem "sumpfnahen Trennboden" ein Trennboden verstanden wird, der in der unteren Hälfte, insbesondere im unteren Drittel, im unteren Viertel oder im unteren Fünftel der Rektifikationskolonne angeordnet ist.

Bei einer "Rektifikationskolonne" handelt es sich im hier verwendeten Sprachgebrauch um eine Trenneinheit, die dafür eingerichtet ist, ein oder mehrere gasförmig oder flüssig oder in Form eines Zweiphasengemischs mit flüssigen und gasförmigen Anteilen, ggf. auch im überkritischen Zustand, eingespeiste Komponentengemische durch Rektifikation zumindest teilweise aufzutrennen, also aus dem oder den Komponentengemischen jeweils Reinstoffe oder zumindest Komponentengemische mit anderer Zusammensetzung zu erzeugen. Die Rektifikation umfasst dabei bekanntermaßen wiederholte Verdampfungs- und Kondensationsvorgänge, insbesondere auf bzw. unter Verwendung von hierfür geeigneten Einbauten, beispielsweise Trennböden oder geordneten oder ungeordneten Packungen. Eine Rektifikationskolonne zum Einsatz im Rahmen der vorliegenden Erfindung weist einen Sumpfverdampfer auf. Hierbei handelt es sich um eine Einrichtung mit einem Wärmetauscher, der beheizt wird und dafür eingerichtet ist, eine im Sumpf der Rektifikationskolonne anfallende flüssige Fraktion, auch als Sumpfflüssigkeit bezeichnet, zu erwärmen. Mittels eines Sumpfverdampfers wird kontinuierlich ein Teil der Sumpfflüssigkeit verdampft und gasförmig in die Rektifikationskolonne zurückgespeist. Eine Rektifikationskolonne zum Einsatz im Rahmen der vorliegenden Erfindung ferner einen Kopfkondensator auf, der in der Rektifikationskolonne aufsteigendes Gas kondensiert und in kondensiertem Zustand auf die Rektifikationskolonne zurückführt.

Zur Auslegung und spezifischen Ausgestaltung von Rektifikationskolonnen und weiteren Trenneinrichtungen sei auf einschlägige Lehrbücher verwiesen (siehe beispielsweise K. Sattler, "Thermische Trennverfahren: Grundlagen, Auslegung, Apparate", 3. Auflage, Wiley-VCH, Weinheim 2001).

Zu Trennverfahren, wie sie spezifisch zur Behandlung von Komponentengemischen eingesetzt werden, die unter Verwendung von Dampfspaltverfahren gebildet werden, insbesondere Trennverfahren, die eine Demethanisierung und eine Deethanisierung umfassen, sei auf den bereits zitierten Artikel "Ethylene" in Ullmann's Encyclopedia of Industrial Chemistry verwiesen. Derartige Trennverfahren unterscheiden sich insbesondere durch die Reihenfolge der jeweiligen Trennschritte. So sind beispielsweise die bereits mehrfach erwähnten Demethanizer-First-Verfahren (auch als Front-End-Demethanizer-Verfahren bezeichnet) und die ebenfalls mehrfach erwähnten Deethanizer-First-Verfahren (auch als Front-End-Deethanizer-Verfahren bezeichnet), aber auch Depropanizer-First-Verfahren (auch als Front-End-Depropanizer-Verfahren bezeichnet) bekannt. Wie auch nachfolgend noch im Detail erläutert, eignet sich die vorliegende Erfindung insbesondere zum Einsatz im Zusammenhang mit Deethanizer-First-Verfahren, aber auch zum Einsatz mit Demethanizer-First-Verfahren oder Depropanizer-First-Verfahren.

Insbesondere können Demethanizer, Deethanizer und Depropanizer, wie sie bereits zuvor erwähnt wurden, als entsprechende Rektifikationskolonnen ausgebildet sein bzw. derartige Rektifikationskolonnen umfassen, die nachfolgend auch als Demethanisierungskolonnen, Deethanisierungskolonnen bzw. Depopanisierungskolonnen bezeichnet werden. Dabei werden im hier verwendeten Sprachgebrauch unter "Demethanizern", "Deethanizern" und "Depropanizern" Anordnungen mit entsprechenden Rektifikationskolonnen verstanden, denen aber auch zusätzliche Apparate, beispielsweise Absorbern bei Deethanizern, zugeordnet sein können. Entsprechendes gilt, wenn davon die Rede ist, dass eine "Demethanisierung", eine "Deethanisierung" oder eine "Depropanisierung" vorgenommen wird. Ist nachfolgend davon die Rede, dass Fraktionen "vom Kopf" oder "aus dem Sumpf" von Demethanizern, Deethanizern und Depropanizern oder entsprechenden Rektifikationskolonnen abgezogen werden können, können diese auch alternativ oder zusätzlich zur Rektifikationskolonne vom Kopf bzw. aus dem Sumpf entsprechender zugeordneter Apparate abgezogen werden.

Gemäß einer ersten bevorzugten Ausgestaltung der vorliegenden Erfindung werden bei der Entfernung von Wasserstoff und Methan in dem oder den zweiten Vortrennschritten auch Ethan und Ethylen zumindest überwiegend entfernt, es wird also ein Deethanizer-First-Verfahren durchgeführt.

Dabei erfolgt die Entfernung von Wasserstoff und Methan in dem oder den zweiten Vortrennschritten, bei der auch Ethan und Ethylen zumindest überwiegend entfernt werden, unter Verwendung einer Deethanisierungskolonne. Im Bereich eines sumpfnahen Bodens einer derartigen Deethanisierungskolonne fällt in diesem Fall das vierte Komponentengemisch an. Eine entsprechende Flüssigkeitist gegenüber dem zweiten Komponentengemisch an Wasserstoff, Methan sowie Kohlenwasserstoffen mit zwei Kohlenstoffatomen abgereichert bzw. wurden entsprechende Komponenten zu einem überwiegenden Anteil entfernt.

Wie mehrfach erwähnt, ist alternativ zu der soeben erläuterten ersten bevorzugten Ausgestaltung der Erfindung auch eine zweite vorteilhafte Ausgestaltung möglich, bei denen bei der Entfernung von Wasserstoff und Methan in dem oder den zweiten Vortrennschritten Ethan und Ethylen zumindest überwiegend nicht entfernt werden. Ethan und Ethylen gehen daher in diesen Ausführungsformen der Erfindung zumindest zum überwiegenden Teil in das vierte Komponentengemisch über. Es kommt also insbesondere ein Demethanizer-First-Verfahren zum Einsatz. Wird zur Entfernung von Wasserstoff und Methan dabei eine entsprechende Rektifikationskolonne eingesetzt, handelt es sich bei dem vierten Komponentengemisch insbesondere um eine Sumpfflüssigkeit einer derartigen Rektifikationskolonne.

Die Entfernung von Wasserstoff und Methan in dem oder den zweiten Vortrennschritten, bei der Ethan und Ethylen zumindest überwiegend nicht entfernt werden, kann also unter Verwendung einer Demethanisierungskolonne durchgeführt werden. Einer derartigen Demethanisierungskolonne oder einem dieser zugeordndeten Apparat wird kopfseitig ein überwiegend oder ausschließlich Wasserstoff und Methan enthaltendes Komponentengemisch entnommen, aus dem Sumpf kann eine überwiegend oder ausschließlich Kohlenwasserstoffe mit zwei und mehr Kohlenstoffatomen enthaltende Sumpfflüssigkeit abgezogen werden. Diese Sumpfflüssigkeit kann insbesondere anschließend einer Deethanisierung unterworfen werden, wie auch nachfolgend noch erläutert.

Mit anderen Worten ist also in einem Demethanizer-First-Verfahren zusätzlich zur Demethanisierungskolonne eine Deethanisierungskolonne vorgesehen. Hierbei kann das dritte Komponentengemisch insbesondere in einen Sumpf oder den Bereich eines sumpfnahen Trennbodens der Demethanisierungskolonne oder in den unteren Bereich, d.h. den Bereich eines sumpfnahen Trennbodens, der Deethanisierungskolonne eingespeist werden. Kommt ein Deethanizer-First-Verfahren zum Einsatz, kann das dritte Komponentengemisch insbesondere in den unteen Bereich der Deethanisierungskolonne eingespeist werden. In beiden Fällen, also in einem Demethanizer-First-Verfahren und in einem Deethanizer-First-Verfahren, kommen ferner Depropanisierungskolonnen zum Einsatz. Auch in einer Depropanisierungskolonne kann alternativ oder zusätzlich eine Einspeisung des dritten Komponentengemischs erfolgen. Entsprechendes gilt auch, wenn ein Depropanizer-First-Verfahren eingesetzt wird. Auch in diesem Fall kann eine Einspeisung des dritten Komponentengemischs in die Depropanisierungskolonne erfolgen.

Insbesondere wird im Rahmen der vorliegenden Erfindung im Rahmen des oder der ersten Vortrennschritte des ersten Komponentengemischs dessen Wasserstoffgehalt auf einen Wert von 0 bis 10 mol%, insbesondere 0,1 bis 5 mol%, beispielsweise 0,2 bis 2 mol% abgereichert. Bei derartigen Wasserstoffgehalten kann das zweite Komponentengemisch, da es in seiner sonstigen Zusammensetzung ausreichend ähnlich einem entsprechenden Fluid aus einem Dampfspaltverfahren ist, auch einer gemeinsamen Trennung zugeführt werden. Wie erwähnt, kann hierbei noch vorhandener Wasserstoff einfach entfernt werden.

Wie erwähnt, umfassen der oder die ersten Vortrennschritte, denen das erste Komponentengemisch unterworfen wird, ferner eine Druckerhöhung, die insbesondere auf einen Absolutdruck von 3 bis 40 bar, insbesondere von 10 bis 30 bar, beispielsweise von 12 bis 30 bar, erfolgt. Das Druckniveau richtet sich dabei andere nach einem Druckniveau, auf der ein Demethanizer oder Deethanizer oder Depropanizer, wie er in dem oder den zweiten Vortrennschritten eingesetzt wird, betrieben wird, also die dort ebenfalls vorgenommene Druckerhöhung im Rahmen des oder der zweiten Vortrennschritte erfolgt. Daher können auf diese Weise die Druckniveaus des dritten und vierten Komponentengemischs in besonders vorteilhafter Weise aneinander angeglichen werden.

Die Wasserstoffabreicherung im Rahmen der Vorbehandlung des ersten Komponentengemischs oder dessen der Vorbehandlung unterworfenen Teils kann insbesondere eine Teilkondensation von Kohlenwasserstoffen mit drei Kohlenstoffatomen nach der erläuterten Druckerhöhung bzw. Verdichtung umfassen. Auf diese Weise wird eine Fraktion gebildet, die überwiegend Kohlenwasserstoffe mit drei Kohlenstoffatomen enthält, in die jedoch auch die anderen genannten Komponenten teilweise übergehen können. Diese Fraktion ist jedenfalls gegenüber dem ersten Komponentengemisch an Wasserstoff abgereichert. Eine entsprechende Kondensation ist besonders vorteilhaft, weil diese im Rahmen der vorliegenden Erfindung, wie nachfolgend erläutert, zumindest teilweise unter Verwendung von Kälte durchgeführt werden kann, welche durch in dem Verfahren vorhandene Prozessströme bereitgestellt werden kann.

Insbesondere kann die Teilkondensation unter Verwendung von Kälte durchgeführt werden, die zumindest teilweise durch Entspannen eines überwiegend Propan enthaltenden Stroms gewonnen werden kann. Bei diesem überwiegend Propan enthaltenden Strom kann es sich beispielsweise um das zweite Trennungsprodukt handeln, das in dem oder den ersten Trennschritten gebildet wird. Dieses zweite Trennungsprodukt kann zur Erzeugung von Kälte entspannt und anschließend in den Prozess zurückgeführt werden, insbesondere in das Verfahren zur Propandehydrierung oder das Dampfspaltverfahren.

Ferner ist es auch möglich, die Teilkondensation unter Verwendung von Kälte durchzuführen, die zumindest teilweise durch Entspannen eines Teils des ersten Komponentengemischs oder dessen dem oder den ersten Vortrennschritten unterworfenen Anteils zu erzeugen. Beispielsweise kann das erste Komponentengemisch oder dessen dem oder den ersten Vortrennschritten unterworfener Anteil dem oder den ersten Vortrennschritten in Form eines Stoffstroms zugeführt werden, der verdichtet wird, und von dem ein Teilstrom stromab der Verdichtung entspannt wird. Der entspannte Teilstrom kann der Verdichtung erneut zugeführt werden, so dass auf diese Weise kontinuierlich Kälte generiert werden kann. Auch sogenannte Coldboxverfahren, wie sie grundsätzlich aus dem Stand der Technik bekannt sind, oder auf anderen Trennprinzipien beruhende Verfahren können im Rahmen der vorliegenden Erfindung eingesetzt werden.

Besonders vorteilhaft ist es, wenn das Verfahren zur Propandehydrierung unter wasserfreien und/oder in kompletter Abwesenheit von Sauerstoff (auch in kovalent gebundener Form und/oder während der Regenerierung) durchgeführt wird. Auf diese Weise wird es möglich, das erste Komponentengemisch derart zu bilden, dass sich in ihm weder Wasser noch sauerstoffhaltige Verbindungen, insbesondere Kohlendioxid, wiederfinden. Auf diese Weise ist es besonders einfach möglich, ein entsprechendes erstes Komponentengemisch dem oder den ersten Vortrennschritten und insbesondere anschließend dem oder den ersten Trennschritten zuzuführen, weil keine Abtrennung dieser Komponenten erforderlich ist. Das erste Komponentengemisch kann, mit anderen Worten, ohne Abtrennung von beispielsweise Wasser und Kohlendioxid bei der Bildung des dritten Komponentengemischs, beispielsweise einer Rektifikationskolonne zugeführt werden, die der Deethanisierung dient, und die typischerweise bei tiefkalten Temperaturen betrieben wird, bei denen Wasser und Kohlendioxid ausfrieren würden.

Wie erwähnt, ist die Zuführung des dritten Komponentengemischs nach dem oder den ersten Vortrennschritten zu dem oder den ersten Trennschritten, in dem oder in denen das dritte Komponentengemisch mit dem vierten Komponentengemisch bzw. einem hieraus gebildeten Komponentengemisch vereinigt wird, dann besonders vorteilhaft, wenn sich die jeweiligen Zusammensetzungen gleichen bzw. um nicht mehr als einen vorbestimmten Umfang unterscheiden.

Es ist daher besonders vorteilhaft, wenn ein Wasserstoffgehalt in dem dritten Komponentengemisch um nicht mehr als 50%, insbesondere um nicht mehr als 25%, beispielsweise um nicht mehr als 10%, von einem Wasserstoffgehalt in dem vierten Komponentengemisch abweicht, und wenn ein Gehalt an Kohlenwasserstoffen mit drei Kohlenstoffatomen, insbesondere Propylen, in dem dritten Komponentengemisch um nicht mehr als 50%, insbesondere um nicht mehr als 25%, beispielsweise um nicht mehr als 10%, von einem Gehalt an Kohlenwasserstoffen mit drei Kohlenstoffatomen, insbesondere Propylen, in dem vierten Komponentengemisch abweicht.

Die vorliegende Erfindung erstreckt sich ferner auf eine Anlage zur Herstellung von Propylen, mit einer ersten Reaktoreinheit, die zum Durchführen eines Verfahrens zur Propandehydrierung unter Erhalt eines ersten Komponentengemischs, das zumindest Wasserstoff, Ethan, Ethylen, Propan und Propylen enthält, bereitgestellt und eingerichtet ist, einer zweiten Reaktoreinheit, die zum Durchführen eines Dampfspaltverfahrens, unter Erhalt eines zweiten Komponentengemischs, das zumindest Wasserstoff, Methan, Ethan, Ethylen, Propan und Propylen enthält, bereitgestellt und eingerichtet ist, und einer ersten Trenneinheit, die zum Bilden eines überwiegend Propan enthaltenden Trennungsprodukts unter Verwendung zumindest eines Teils des Propans des ersten und, gemäß einer bevorzugten Ausführungsform auch des zweiten, Komponentengemischs und unter Einsatz eines oder mehrerer erster Trennschritte bereitgestellt und eingerichtet ist, wobei Mittel bereitgestellt sind, die dafür eingerichtet sind, dem oder den Propanabtrennschritten zumindest einen Teil des ersten Komponentengemischs und, gemäß der bevorzugten Ausführungsform auch zumindest einen Teil des zweiten, Komponentengemischs zuzuführen.

Erfindungsgemäß zeichnet sich eine derartige Anlage aus durch Mittel, die dafür bereitgestellt und eingerichtet sind, das überwiegend Propan enthaltenden Trennungsprodukt zumindest zum Teil in das Dampfspaltverfahren zurückzuführen.

Zu Merkmalen und Vorteilen der erfindungsgemäß vorgeschlagenen Anlage sei auf die zuvor bereits bezüglich des erläuterten Prozesses und seiner vorteilhaften Ausgestaltung in erläuterten Merkmale und Vorteile ausdrücklich verwiesen. Entsprechendes gilt insbesondere für eine Anlage gemäß einer besonders bevorzugten Ausführungsformen der vorliegenden Erfindung, welche Mittel aufweist, die zur Durchführung eines entsprechenden Verfahrens eingerichtet sind.

Die Erfindung erstreckt sich ferner auf ein Verfahren zum Umrüsten einer Anlage, die zur Durchführung eines Dampfspaltverfahrens unter Verwendung einer Anzahl von Anlagenkomponenten wie Spaltöfen, Aufbereitungseinrichtungen und Trennapparaten eingerichtet ist, wobei der Anlage vor der Umrüstung ein Kohlenwasserstoffe enthaltendes Einsatzgemisch mit einer ersten Zusammensetzung zugeführt wird. Die Umrüstung umfasst erfindungsgemäß, der Anlage anstelle des Kohlenwasserstoffe enthaltenden Einsatzgemischs mit der ersten Zusammensetzung ein Kohlenwasserstoffe enthaltendes Einsatzgemisch mit einer zweiten, abweichenden Zusammensetzung zuzuführen, und eine oder mehrere der Anlagenkomponenten anstatt für das Dampfspaltverfahren für ein Verfahren zur Propandehydrierung zu nutzen, also freiwerdende Kapazitäten entsprechend umzuwidmen.

Beispielhaft sei hier eine Änderung des Einsatzgemisches des Dampfspaltverfahrens von schwereren Kohlenwasserstoffen, beispielsweise überwiegend Naphta, hin zu leichteren Kohlenwaserstoffen, beispielsweise Ethan und/oder Propan sowie Butan, zu nennen. Während gewisse Anlagenteile zur Verarbeitung des gesamten Produktgases, wie beispielsweise der Rohgasverdichter, sowie Anlagenteile zur Verarbeitung der leichten Produktfraktion, wie beispielsweise der Demethanizer, nach der Änderung des Einsatzgemisches voraussichtlich gleich stark oder sogar höher als zuvor belastet sind, werden andere Anlagenteile, beispielsweise zur Verarbeitung von schwereren Produktfraktionen, entlastet. Diese entlasteten Anlagenteile können den Depropanizer sowie sämtliche Anlagenteile zur Verarbeitung einer Fraktion mit Kohlenwasserstoffen mit drei Kohlenstoffatomen einschließlich einer Hydrierung und eines Splitters, umfassen. Diese Anlagenteile können dann zusätzlich für ein Verfahren zur Propandehydrierung genutzt werden. Hierbei ist die in der vorliegenden Erfindung beschriebene Vorgehensweise der vorangehenden Wasserstoffentfernung aus dem Produktgas der Propandehydrierung insbesondere von Vorteil, da durch diese Vorgehensweise die stark ausgelasteten bestehenden Anlagenteile des Dampfspaltverfahrens, wie beispielsweise der Demethanizer, nicht noch stärker belastet werden.

Erfindungsgemäß wird nach der Umrüstung ein Prozess durchgeführt, wie er zuvor beschrieben wurde, und/oder es wird mittels der Umrüstung eine entsprechende Anlage bereitgestellt. Auf diese Weise kann der bereits eingangs erwähnte Vorteil erzielt werden, dass entsprechende Produkte einer Propandehydrierung zusammen mit den Produkten des Dampfspaltverfahrens aufzureinigen und auf eine separate Aufreinigung zu verzichten.

Die Erfindung wird nachfolgend unter Bezugnahme auf die beigefügten Zeichnungen näher erläutert, in der eine bevorzugte Ausführungsformen der vorliegenden Erfindung gegenüber dem Stand der Technik erläutert ist.

### Kurze Beschreibung der Zeichnungen

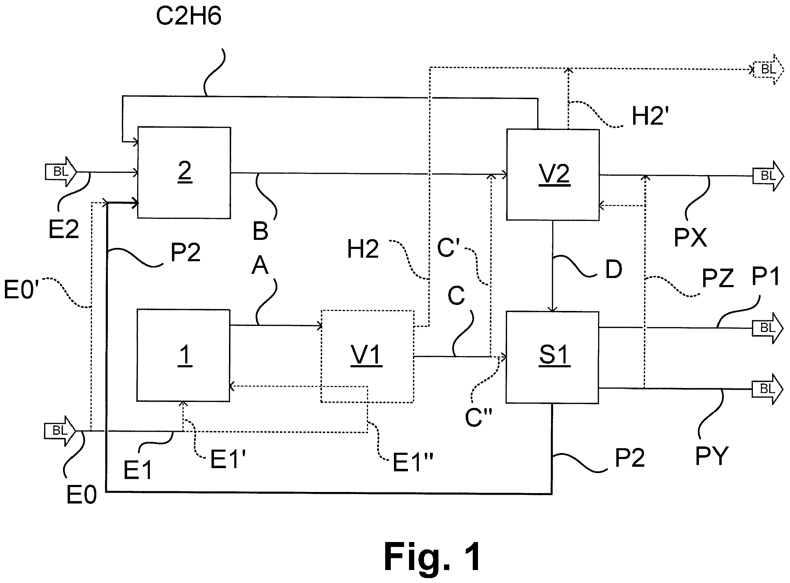

Figur 1 veranschaulicht einen gemäß einer Ausführungsform der Erfindung ausgestalteten Prozess in stark vereinfachter, schematischer Darstellung.

In den Figuren sind einander baulich und/oder funktionell entsprechende Elemente mit identischen Bezugszeichen angegeben und werden der Übersichtlichkeit halber nicht wiederholt erläutert.

### Ausführliche Beschreibung der Zeichnungen

In Figur 1 ist ein erfindungsgemäß ausgestalteter Prozess in stark vereinfachter, schematischer Darstellung veranschaulicht und insgesamt mit 10 bezeichnet.

Der Prozess 10 umfasst ein Verfahren 1 zur Propandehydrierung, ein Dampfspaltverfahren 2, einen oder mehrere erste Vortrennschritte V1, einen oder mehrere zweite Vortrennschritte V2, sowie einen oder mehrere Propanabtrennschritte S1. Der oder die ersten Vortrennschritte V1, der oder die zweiten Vortrennschritte V2, der oder die Propanabtrennschritte S1 und beliebige weitere, hier nicht gesondert veranschaulichte Trennschritte können dabei jeweils beliebig gruppiert und beispielsweise in entsprechenden Anlagenkomponenten zusammengefasst sein. Dem Verfahren 1 zur Propandehydrierung wird im dargestellten Beispiel ein erster Einsatzstrom E1 zugeführt, der insbesondere Propan umfassen kann, jedoch kein rückgeführtes Propan. Der erste Einsatzstrom E1 wird vielmehr ausschließlich unter Verwendung eines Ausgangsstroms E0 gebildet, der dem Prozess 10 von der Anlagengrenze BL zugeführt wird. Ein Teil dieses Ausgangsstroms E0 kann, wie in Form eines gestrichelten Stoffstroms E0' veranschaulicht, auch dem Dampfspaltverfahren 2 zugeführt werden. Der erste Einsatzstrom E1 wird im dargestellten Beispiel in zwei Teilströme E1' und E1" aufgeteilt, wobei der Teilstrom E1' dem Verfahren 1 zur Propandehydrierung direkt zugeführt wird und der Teilstrom E" zunächst in dem oder den ersten Vortrennschritten V1 genutzt wird. Der Teilstrom E1" kann dabei in dem oder den ersten Vortrennschritten V1 beispielsweise zur Erzeugung von Kälte entspannt und erst anschließend dem Verfahren 1 zur Propandehydrierung zugeführt werden. Abweichend zur Darstellung der Figur 1 kann der Einsatzstrom E1 aber auch vollständig wie hier bezüglich des Teilstroms E1' veranschaulicht behandelt werden, also direkt dem Verfahren 1 zur Propandehydrierung zugeführt werden.

Das Verfahren 1 zur Propandehydrierung wird in grundsätzlich bekannter Weise durchgeführt, so dass in diesem ein erstes Komponentengemisch A gebildet wird, das zumindest Wasserstoff, Ethan, Ethylen, Propan und Propylen enthält, und das aus dem Verfahren 1 zur Propandehydrierung in Form eines entsprechenden Stoffstroms ausgeführt werden kann. Das Verfahren 1 zur Propandehydrierung kann insbesondere unter Verwendung eines oder mehrerer geeigneter Reaktoren, die in fachüblicher Weise ausgebildet sein können, durchgeführt werden.

Das erste Komponentengemisch A bzw. der entsprechende Stoffstrom wird im dargestellten Beispiel zumindest teilweise dem oder den ersten Vortrennschritten V1 zugeführt, in dem oder denen das erste Komponentengemisch A bzw. der entsprechende Stoffstrom einer Druckerhöhung und einer zumindest teilweisen Entfernung von Wasserstoff unterworfen wird. Wie oben erwähnt, kann dies in grundsätzlich bekannter Weise erfolgen. Insbesondere kann das erste Komponentengemisch A bzw. der entsprechende Stoffstrom in dem oder den ersten Vortrennschritten V1 verflüssigt werden. Abgetrennter Wasserstoff ist in Form eines mit H2 bezeichneten Stoffstroms veranschaulicht. Auch eine zumindest teilweise Entfernung von Kohlenwasserstoffen mit zwei Kohlenstoffatomen ist möglich, aber optional, wie in Figur 1 nicht gesondert veranschaulicht. Auf diese Weise wird ein Komponentengemisch erhalten, das hier auch als drittes Komponentengemisch C bezeichnet wird, und das in Form eines entsprechenden Stoffstroms aus dem oder den ersten Vortrennschritten V1 ausgeführt werden kann. Mögliche Wasserstoffgehalte des dritten Komponentengemischs C bzw. des entsprechenden Stoffstroms wurden bereits oben erläutert. Insbesondere enthält das dritte Komponentengemisch C stromab des oder der ersten Vortrennschritte V1 noch Kohlenwasserstoffe mit drei Kohlenstoffatomen sowie geringere Mengen an anderen Komponenten, beispielsweise Kohlenwasserstoffe mit zwei Kohlenwasserstoffatomen, soweit noch nicht entfernt, und Kohlenwasserstoffe mit vier Kohlenstoffatomen, die in dem Verfahren 1 zur Propandehydrierung als Nebenprodukte gebildet werden. Sofern im ersten Komponentengemisch A auch andere Wasser und Kohlendioxid enthalten sind, können diese auch in dem oder den ersten Vortrennschritten V1 entfernt werden.

Dem Dampfspaltverfahren 2, das ebenfalls in fachüblicher Weise, beispielsweise auch unter Verwendung mehrerer Spaltöfen, durchgeführt werden kann, wird im dargestellten Beispiel ein kohlenwasserstoffreicher Einsatz in Form eines Stoffstroms E2 zugeführt, der von der Anlagengrenze BL zugeführt wird. Der kohlenwasserstoffreiche Einsatz kann insbesondere Naphtha und leichtere Kohlenwasserstoffe, aber auch schwere Kohlenwasserstoffe, umfassen. Insbesondere kann der kohlenwasserstoffreiche Einsatz auch paraffinische Kohlenwasserstoffe mit zwei, drei und vier Kohlenstoffatomen umfassen, insbesondere Ethan, Propan und Butan. Dem Dampfspaltverfahren 2 insgesamt oder unterschiedlichen Spaltöfen, die in dem Dampfspaltverfahren 2 eingesetzt werden, können auch unterschiedliche Kohlenwasserstoffeinsätze zugeführt und dort unter unterschiedlichen Spaltbedingungen bearbeitet werden. Im dargestellten Beispiel werden dem Dampfspaltverfahren 2 zusätzlich der erwähnte Teilstrom E0' des Ausgangsstroms E0, ein überwiegend Propan enthaltendes Trennungsprodukt P2 des oder der Propanabtrennschritte S1 und ein weiterer zurückgeführter Strom C2H6 zugeführt, der überwiegend oder ausschließlich Ethan enthält. Bis auf die Rückführung des überwiegend Propan enthaltenden Trennungsprodukts P2 ist die Zuführung bzw. Rückführung der anderen dargestellten Ströme im Rahmen der vorliegenden Erfindung völlig optional.

In dem Dampfspaltverfahren 2 werden die Kohlenwasserstoffe des oder der kohlenwasserstoffreichen Einsätze zumindest teilweise umgesetzt, so dass ein zweites Komponentengemisch B erhalten wird, das zumindest Wasserstoff, Methan, Ethan, Ethylen, Propan und Propylen enthält. Das zweite Komponentengemisch B kann aus dem Dampfspaltverfahren 2 in Form eines entsprechenden Stoffstroms abgezogen und anschließend zumindest zum Teil dem einem oder den mehreren zweiten Vortrennschritten V2 zugeführt werden. Die Zusammensetzung des zweiten Komponentengemischs B bzw. des entsprechenden Stoffstroms richtet sich maßgeblich nach dem dem Dampfspaltverfahren 2 zugeführten kohlenwasserstoffreichen Einsatz.

Wie bereits mehrfach erläutert, kann bzw. können der oder die zweiten Vortrennschritte V2 insbesondere eine Demethanisierung und/oder eine Deethanisierung umfassen. Beispielsweise kann ein Demethanizer-First-Verfahren oder ein Deethanizer-First-Verfahren zum Einsatz kommen. Beide Varianten wurden zuvor bereits erläutert und sind aus dem Stand der Technik grundsätzlich bekannt. Sie werden daher an dieser Stelle nicht erneut erläutert. In dem oder den zweiten Vortrennschritten können dabei neben Wasserstoff, wie hier in Form eines Stoffstroms H2' veranschaulicht, auch der erwähnte Stoffstrom C2H6, der überwiegend oder ausschließlich Ethan enthält, sowie ein oder mehrere Produktströme, hier mit PX zusammengefasst, gebildet werden. Der oder die Produktströme PX können an die Anlagengrenze BL geführt werden. Der wasserstoffreiche Stoffstrom H2' kann mit dem wasserstoffreichen Stoffstrom H2 vereinigt und zusammen mit diesem an die Anlagengrenze BL geführt werden. Zusammen mit dem wasserstoffreichen Stoffstrom H2' kann insbesondere auch Methan ausgeleitet werden.
Ungeachtet des spezifisch durchgeführten Verfahrens wird jedoch in beiden Fällen durch die Verwendung des oder der zweiten Vortrennschritte V2 ein Komponentengemisch D gebildet, das hier als viertes Komponentengemisch bezeichnet wird, und das gegenüber dem zweiten Komponentengemisch B zumindest an Wasserstoff und an Methan abgereichert ist bzw. das dadurch gebildet wird, dass zumindest Wasserstoff von Methan zumindest teilweise aus dem zweiten Komponentengemisch B entfernt werden. Dieses vierte Komponentengemisch D wird dem oder den Propanabtrennschritten S1 zugeführt, in dem oder denen das bereits erwähnte, überwiegend Propan enthaltende Trennungsprodukt P2 gebildet wird. Ferner kann im dargestellten Beispiel in dem oder den Propanabtrennschritten S1 auch ein überwiegend oder ausschließlich Propylen enthaltendes Trennungsprodukt P1 gebildet und an die Anlagengrenze BL geführt werden. Ein oder mehrere weitere Trennungsprodukte, hiermit PY zusammengefasst, können ebenfalls in dem oder den Propanabtrennschritten S1 abgetrennt und an die Anlagengrenze BL geführt werden. Je nach der spezifischen Auslegung des erfindungsgemäßen Verfahrens können weitere Trennschritte, beispielsweise eine Deethanisierung oder eine Depropanisierung, als Teil des oder der zweiten Vortrennschritte V2 oder als Teil des oder der Propanabtrennschritte S1 ausgebildet sein.

Das dritte Komponentengemisch C kann dem oder den zweiten Vortrennschritten V2 und/oder dem oder den Propanabtrennschritten S1 zugeführt werden. Beispielsweise kann das dritte Komponentengemisch in den Bereich eines sumpfnahen Trennbodens einer in dem oder den zweiten Vortrennschritten V2 eingesetzten Demethanisierungskolonne oder Deethanisierungskolonne und/oder in eine Depropanisierungskolonne, die Teil des oder der zweiten Vortrennschritte V2 und oder des oder der Propanabtrennschritte S1 sein kann. Entsprechende Alternativen sind in Form der Stoffströme C' und C" veranschaulicht. Stoffströme können auch, wie hier in Form des Stoffstroms PZ veranschaulicht, von dem oder den Propanabtrennschritten S1 in den oder die zweiten Vortrennschritte V2 zurückgeführt werden.

## Patentansprüche

1. Prozess (10) zur Herstellung von Propylen, der umfasst:
- Durchführen eines Verfahrens (1) zur Propandehydrierung unter Erhalt eines ersten Komponentengemischs (A), das zumindest Wasserstoff, Ethan, Ethylen, Propan und Propylen enthält,
- Durchführen eines Dampfspaltverfahrens (2) unter Erhalt eines zweiten Komponentengemischs (B), das zumindest Wasserstoff, Methan, Ethan, Ethylen, Propan und Propylen enthält,
- Bilden eines überwiegend Propan enthaltenden Trennungsprodukts (P2) unter Verwendung zumindest eines Teils des Propans des ersten Komponentengemischs (A) und unter Einsatz eines oder mehrerer Propanabtrennschritte (S1),
- wobei dem oder den Propanabtrennschritten (S1) zumindest ein Teil des ersten Komponentengemischs (A) zugeführt wird,
**dadurch gekennzeichnet,**
- **dass** das überwiegend Propan enthaltende Trennungsprodukt (P2) zumindest zum Teil in das Dampfspaltverfahrens (2) zurückgeführt wird.

2. Prozess (10) nach Anspruch 1, bei dem das Bilden des überwiegend Propan enthaltenden Trennungsprodukts (P2) unter Einsatz eines oder mehrerer Propanabtrennschritte (S1) ferner unter Verwendung zumindest eines Teils des Propans des zweiten Komponentengemischs (B) erfolgt, wobei dem oder den Propanabtrennschritten (S1) ferner zumindest ein Teil des zweiten Komponentengemischs (B) zugeführt wird.

3. Prozess (10) nach Anspruch 1 oder 2, bei dem das überwiegend Propan enthaltende Trennungsprodukt (P2) nicht in das Verfahren (1) zur Propandehydrierung zurückgeführt wird.

4. Prozess (10) nach einem der vorstehenden Ansprüche, bei welchem dem Verfahren (1) zur Propandehydrierung lediglich Propan zugeführt wird, das weder aus dem ersten Komponentengemisch (A) noch aus dem zweiten Komponentengemisch (B) abgetrennt wurde.

5. Prozess (10) nach einem der vorstehenden Ansprüche, bei dem das überwiegend Propan enthaltende Trennungsprodukt überwiegend Propan und ferner von 0,1 bis 25 Volumenprozent einfach und mehrfach ungesättigte C3 Kohlenwasserstoffe und/oder schwerere Kohlenwasserstoffe enthält.

6. Prozess (10) nach einem der vorstehenden Ansprüche, der ferner umfasst
- Bilden eines überwiegend oder ausschließlich Propylen enthaltenden Trennungsprodukts (P1) unter Verwendung zumindest eines Teils des Propylens des ersten und des zweiten Komponentengemischs (A, B) und unter Einsatz des oder der Propanabtrennschritte (S1),
- Bilden eines zumindest überwiegend Ethylen enthaltenden Trennungsprodukts (P3) unter Verwendung zumindest eines Teils des Ethylens des ersten und des zweiten Komponentengemischs (A, B) und unter Einsatz eines oder mehrerer weiterer Trennschritte, und
- Bilden eines zumindest überwiegend Ethan enthaltenden Trennungsprodukts (P4) unter Verwendung zumindest eines Teils des Ethans des ersten und des zweiten Komponentengemischs (A, B) und unter Einsatz des oder der weiteren Trennschritte.

7. Prozess (10) nach einem der vorstehenden Ansprüche, der umfasst
- dass zumindest ein Teil des ersten Komponentengemischs (A) unter Erhalt eines dritten Komponentengemischs (C) einem oder mehreren ersten Vortrennschritten (V1) unterworfen wird, der oder die eine Druckerhöhung und eine zumindest teilweise Entfernung von Wasserstoff umfassen,
- dass zumindest ein Teil des zweiten Komponentengemischs (B) unter Erhalt eines vierten Komponentengemischs (D) einem oder mehreren zweiten Vortrennschritten (V2) unterworfen wird, der oder die eine Druckerhöhung, eine zumindest teilweise Entfernung von Wasserstoff und eine zumindest teilweise Entfernung von Methan umfassen, und
- dass zumindest ein Teil des dritten Komponentengemischs (C) gemeinsam mit zumindest einem Teil des vierten Komponentengemischs (D) dem oder den Propanabtrennschritten (S1) unterworfen wird und/oder
- dass zumindest ein Teil des dritten Komponentengemischs (C) zusammen mit dem zweiten Komponentengemisch (B) unter Bildung des vierten Komponentengemischs (D) dem oder den zweiten Vortrennschritten (V2) unterworfen wird und das vierte Komponentengemisch (D) dem oder den Propanabtrennschritten (S1) unterworfen wird.

8. Prozess (10) nach Anspruch 7, bei dem bei der Entfernung von Wasserstoff und Methan in dem oder den zweiten Vortrennschritten (V2) auch Ethan und Ethylen zumindest überwiegend entfernt werden, wobei die Entfernung von Wasserstoff und Methan in dem oder den zweiten Vortrennschritten (V2), bei der auch Ethan und Ethylen zumindest überwiegend entfernt werden, unter Verwendung einer Deethanisierungskolonne durchgeführt wird.

9. Prozess (10) nach Anspruch 7, bei dem bei der Entfernung von Wasserstoff und Methan in dem oder den zweiten Vortrennschritten (V2) Ethan und Ethylen zumindest überwiegend nicht entfernt werden, wobei die Entfernung von Wasserstoff und Methan in dem oder den zweiten Vortrennschritten (V2), bei der Ethan und Ethylen zumindest überwiegend nicht entfernt werden, unter Verwendung einer Demethanisierungskolonne durchgeführt wird.

10. Prozess (10) nach Anspruch 7, bei dem in dem oder den zweiten Vortrennschritten (V2) und/oder in dem oder den Propanabtrennschritten (S1) eine Demethanisierungskolonne und/oder eine Deethanisierungskolonne und/oder eine Depropanisierungskolonne verwendet wird, wobei das dritte Komponentengemisch zumindest teilweise in flüssigem Zustand in den unteren Bereich der Demethanisierungskolonne und/oder der Deethanisierungskolonne und/oder der Depropanisierungskolonne eingespeist wird.

11. Prozess (10) nach einem der Ansprüche 7 bis 10, bei dem in dem oder den ersten Vortrennschritten (V1) des ersten Komponentengemischs dessen Wasserstoffgehalt auf einen Wert von 0 bis 10 mol%, insbesondere 0,1 bis 5 mol%, beispielsweise 0,2 bis 2 mol% abgereichert wird.

12. Prozess (10) nach einem der vorstehenden Ansprüche, bei dem der oder die ersten Vortrennschritte, denen das erste Komponentengemisch unterworfen wird, eine Druckerhöhung auf einen Absolutdruck von 3 bis 40 bar, insbesondere von 10 bis 30 bar, beispielsweise von 12 bis 30 bar, umfassen, wobei insbesondere in dem oder den ersten Vortrennschritten nach der Druckerhöhung eine zumindest teilweise Kondensation von schwerer als Wasserstoff siedenden Komponenten durchgeführt wird.

13. Anlage zur Herstellung von Propylen mit:
- einer ersten Reaktoreinheit, die zum Durchführen eines Verfahrens (1) zur Propandehydrierung unter Erhalt eines ersten Komponentengemischs (A), das zumindest Wasserstoff, Ethan, Ethylen, Propan und Propylen enthält, bereitgestellt und eingerichtet ist,
- einer zweiten Reaktoreinheit, die zum Durchführen eines Dampfspaltverfahrens (2) unter Erhalt eines zweiten Komponentengemischs (B), das zumindest Wasserstoff, Methan, Ethan, Ethylen, Propan und Propylen enthält, bereitgestellt und eingerichtet ist, und
- einer Trenneinheit, die zum Bilden eines überwiegend Propan enthaltenden Trennungsprodukts (P2) unter Verwendung zumindest eines Teils des Propans des ersten Komponentengemischs (A) und unter Einsatz eines oder mehrerer Propanabtrennschritte (S1) bereitgestellt und eingerichtet ist,
- wobei Mittel bereitgestellt sind, die dafür eingerichtet sind, dem oder den Propanabtrennschritten (S1) zumindest einen Teil des ersten Komponentengemischs (A) zuzuführen,
**gekennzeichnet durch**
- Mittel, die dafür bereitgestellt und eingerichtet sind, das überwiegend Propan enthaltende Trennungsprodukt (P2) zumindest zum Teil in das Dampfspaltverfahren (2) zurückzuführen.

14. Verfahren zum Umrüsten einer Anlage, die zur Durchführung eines Dampfspaltverfahrens unter Verwendung einer Anzahl von Anlagenkomponenten eingerichtet ist, wobei der Anlage vor der Umrüstung ein Kohlenwasserstoffe enthaltendes Einsatzgemisch mit einer ersten Zusammensetzung zugeführt wird, **dadurch gekennzeichnet, dass** die Umrüstung umfasst, der Anlage anstelle des Kohlenwasserstoffe enthaltenden Einsatzgemischs mit der ersten Zusammensetzung ein Kohlenwasserstoffe enthaltendes Einsatzgemisch mit einer zweiten, abweichenden Zusammensetzung zuzuführen, und eine oder mehrere der Anlagenkomponenten anstatt für das Dampfspaltverfahren für ein Verfahren zur Propandehydrierung zu nutzen, wobei nach der Umrüstung ein Prozess (10) nach einem der Ansprüche 1 bis 12 eingesetzt oder mittels der Umrüstung eine Anlage nach Anspruch 13 bereitgestellt wird.

## Claims

1. Process (10) for producing propylene, comprising:
- carrying out a process (1) for propane dehydrogenation to obtain a first constituent mixture (A) containing at least hydrogen, ethane, ethylene, propane and propylene,
- carrying out a steam cracking process (2) to obtain a second constituent mixture (B) containing at least hydrogen, methane, ethane, ethylene, propane and propylene,
- forming a predominantly propane-containing separation product (P2) by using at least a portion of the propane of the first constituent mixture (A) and using one or more propane separation steps (S1),
- wherein at least a portion of the first constituent mixture (A) is fed to the propane separation step or steps (S1),
**characterized in**
- **that** the separation product (P2) comprising predominantly propane is at least partially recycled into the steam cracking process (2).

2. Process (10) according to claim 1, wherein the forming of the predominantly propane-containing separation product (P2) using one or more propane separation steps (S1) further occurs using at least a portion of the propane of the second constituent mixture (B), wherein at least a portion of the second constituent mixture (B) is further fed to the propane separation step or steps (S1).

3. Process (10) according to claim 1 or 2, wherein the predominantly propane-containing separation product (P2) is not recycled into the process (1) for propane dehydrogenation.

4. Process (10) according to any one of the preceding claims, wherein only propane is fed to the process (1) for propane dehydrogenation which was separated neither from the first constituent mixture (A) nor from the second constituent mixture (B).

5. Process (10) according to any one of the preceding claims, wherein the predominantly propane-containing separation product contains predominantly propane and further from 0.1 to 25% by volume mono- and polyunsaturated C3 hydrocarbons and/or heavier hydrocarbons.

6. Process (10) according to any one of the preceding claims, further comprising
- forming a separation product (P1) containing predominantly or exclusively propylene using at least a portion of the propylene of the first and of the second constituent mixture (A, B) and using the propane separation step or steps (S1),
- forming a separation product (P3) containing at least predominantly ethylene using at least a portion of the ethylene of the first and of the second constituent mixture (A, B) and using one or more further separation steps, and
- forming a separation product (P4) containing at least predominantly ethane using at least a portion of the ethane of the first and of the second constituent mixture (A, B) and using the further separation step or steps.

7. Process (10) according to any one of the preceding claims, comprising
- that at least a portion of the first constituent mixture (A) is subjected to one or more first pre-separation steps (V1) comprising a pressure increase and an at least partial removal of hydrogen to obtain a third constituent mixture (C),
- that at least a portion of the second constituent mixture (B) is subjected to one or more second pre-separation steps (V2) comprising a pressure increase, an at least partial removal of hydrogen and an at least partial removal of methane to obtain a fourth constituent mixture (D), and
- that at least a portion of the third constituent mixture (C) is subjected to the propane separation step or steps (S1) together with at least a portion of the fourth constituent mixture (D) and/or
- that at least a portion of the third constituent mixture (C) is subjected to the second pre-separation step or steps (V2) together with the second constituent mixture (B) to form the fourth constituent mixture (D) and the fourth constituent mixture (D) is subjected to the propane separation step or steps (S1).

8. Process (10) according to claim 7, wherein during the removal of hydrogen and methane in the second pre-separation step or steps (V2) ethane and ethylene are also at least predominantly removed, wherein the removal of hydrogen and methane in the second pre-separation step or steps (V2), in which ethane and ethylene are also at least predominantly removed, is carried out using a deethanizer column.

9. Process (10) according to claim 7, wherein during the removal of hydrogen and methane in the second pre-separation step or steps (V2), ethane and ethylene are at least predominantly not removed, wherein the removal of hydrogen and methane in the second pre-separation step or steps (V2), in which ethane and ethylene are at least predominantly not removed, is carried out using a demethanizer column.

10. Process (10) according to claim 7, wherein a demethanizer column and/or a deethanizer column and/or a depropanizer column is used in the second pre-separation step or steps (V2) and/or in the propane separation step or steps (S1), wherein the third constituent mixture is fed at least partially in liquid state into the lower region of the demethanizer column and/or the deethanizer column and/or the depropanizer column.

11. Process (10) according to any one of claims 7 to 10, wherein in the first pre-separation step or steps (V1) of the first constituent mixture its hydrogen content is depleted to a value of 0 to 10 mol%, in particular 0.1 to 5 mol%, for example 0.2 to 2 mol%.

12. Process (10) according to any one of the preceding claims, wherein the first pre-separation step or steps to which the first constituent mixture is subjected comprise a pressure increase to an absolute pressure of 3 to 40 bar, in particular of 10 to 30 bar, for example of 12 to 30 bar, wherein an at least partial condensation of constituents boiling heavier than hydrogen is carried out in particular in the first pre-separation step or steps after the pressure increase.

13. Plant for producing propylene, comprising:
- a first reactor unit provided and configured to carry out a process (1) for propane dehydrogenation to obtain a first constituent mixture (A) containing at least hydrogen, ethane, ethylene, propane and propylene,
- a second reactor unit provided and configured to carry out a steam cracking process (2) to obtain a second constituent mixture (B) containing at least hydrogen, methane, ethane, ethylene, propane and propylene, and
- a separation unit provided and configured to form a predominantly propane-containing separation product (P2) using at least a portion of the propane of the first constituent mixture (A) and using one or more propane separation steps (S1),
- wherein means are provided which are configured to supply at least a portion of the first constituent mixture (A) to the propane separation step or steps (S1),
**characterized by**
- means which are provided and configured to at least partially recycle the predominantly propane-containing separation product (P2) into the steam cracking process (2).

14. Method for retrofitting a plant configured to carry out a steam cracking process using a number of plant components, wherein before the retrofitting a hydrocarbon-containing feed mixture having a first composition is fed to the plant, **characterized in that** the retrofitting comprises feeding to the plant a hydrocarbon-containing feed mixture having a second, different composition instead of the hydrocarbon-containing feed mixture having the first composition, and using one or more of the plant components for a process for propane dehydrogenation instead of for the steam cracking process, wherein after the retrofitting a process (10) according to any one of claims 1 to 12 is used or a plant according to claim 13 is provided by means of retrofitting.

## Revendications

1. Procédé (10) de production de propylène comprenant :
- la mise en œuvre d'un procédé (1) de déshydrogénation du propane avec obtention d'un premier mélange de composants (A), lequel contient au moins de l'hydrogène, de l'éthane, de l'éthylène, du propane et du propylène,
- la mise en œuvre d'un procédé de craquage à la vapeur (2) avec obtention d'un deuxième mélange de composants (B), lequel contient au moins de l'hydrogène, du méthane, de l'éthane, de l'éthylène, du propane et du propylène,
- la formation d'un produit de séparation (P2) contenant essentiellement du propane à l'aide d'au moins une partie du propane du premier mélange de composants (A) et au moyen d'une ou de plusieurs étapes de séparation du propane (S1),
- la ou les étapes de séparation du propane (S1) étant alimentées en au moins une partie du premier mélange de composants (A),
**caractérisé**
- **en ce que** le produit de séparation (P2) contenant essentiellement du propane est au moins partiellement recyclé dans le procédé de craquage à la vapeur (2).

2. Procédé (10) selon la revendication 1, selon lequel la formation du produit de séparation (P2) contenant essentiellement du propane au moyen d'une ou de plusieurs étapes de séparation du propane (S1) est effectuée en outre à l'aide d'au moins une partie du propane du deuxième mélange de composants (B), la ou les étapes de séparation du propane (S1) étant en outre alimentées en au moins une partie du deuxième mélange de composants (B).

3. Procédé (10) selon la revendication 1 ou 2, selon lequel le produit de séparation (P2) contenant essentiellement du propane n'est pas recyclé dans le procédé (1) de déshydrogénation du propane.

4. Procédé (10) selon l'une quelconque des revendications précédentes, selon lequel le procédé (1) de déshydrogénation du propane est uniquement alimenté en propane lequel n'a été séparé ni du premier mélange de composants (A) ni du deuxième mélange de composants (B).

5. Procédé (10) selon l'une quelconque des revendications précédentes, selon lequel le produit de séparation contenant essentiellement du propane contient essentiellement du propane et en outre de 0,1 à 25 % en volume d'hydrocarbures monoinsaturés et polyinsaturés en C3 et/ou d'hydrocarbures plus lourds.

6. Procédé (10) selon l'une quelconque des revendications précédentes comprenant en outre
- la formation d'un produit de séparation (P1) contenant essentiellement ou exclusivement du propylène à l'aide d'au moins une partie du propylène du premier et du deuxième mélange de composants (A, B) et au moyen de la ou des étapes de séparation du propane (S1),
- la formation d'un produit de séparation (P3) contenant au moins essentiellement de l'éthylène à l'aide d'au moins une partie de l'éthylène du premier et du deuxième mélange de composants (A, B) et au moyen d'une ou de plusieurs autres étapes de séparation et
- la formation d'un produit de séparation (P4) contenant au moins essentiellement de l'éthane, à l'aide d'au moins une partie de l'éthane du premier et du deuxième mélange de composants (A, B) et au moyen de la ou des autres étapes de séparation.

7. Procédé (10) selon l'une quelconque des revendications précédentes comprenant
- la soumission d'au moins une partie du premier mélange de composants (A), avec obtention d'un troisième mélange de composants (C), à une ou plusieurs étapes de préséparation (V1), laquelle ou lesquelles comprennent une augmentation de pression et une élimination au moins partielle de l'hydrogène,
- la soumission d'au moins une partie du deuxième mélange de composants (B), avec obtention d'un quatrième mélange de composants (D), à une ou plusieurs secondes étapes de préséparation (V2), laquelle ou lesquelles comprennent une augmentation de pression, une élimination au moins partielle de l'hydrogène et une élimination au moins partielle du méthane et
- la soumission d'au moins une partie du troisième mélange de composants (C) conjointement avec au moins une partie du quatrième mélange de composants (D) à la ou aux étapes de séparation du propane (S1) et/ou
- la soumission d'au moins une partie du troisième mélange de composants (C) conjointement avec le deuxième mélange de composants (B), avec formation du quatrième mélange de composants (D), à la ou aux secondes étapes de préséparation (V2) et la soumission du quatrième mélange de composants (D) à la ou aux étapes de séparation du propane (S1).

8. Procédé (10) selon la revendication 7, selon lequel, lors de l'élimination de l'hydrogène et du méthane dans la ou les secondes étapes de préséparation (V2), de l'éthane et de l'éthylène sont également au moins essentiellement éliminés, l'élimination de l'hydrogène et du méthane dans la ou les secondes étapes de préséparation (V2), selon laquelle de l'éthane et de l'éthylène sont également au moins essentiellement éliminés, étant mise en œuvre à l'aide d'une colonne de déséthanisation (DE).

9. Procédé (10) selon la revendication 7, selon lequel, lors de l'élimination de l'hydrogène et du méthane dans la ou les secondes étapes de préséparation (V2), de l'éthane et de l'éthylène ne sont pas au moins essentiellement éliminés, l'élimination de l'hydrogène et du méthane dans la ou les secondes étapes de préséparation (V2), selon laquelle ou lesquelles de l'éthane et de l'éthylène ne sont pas au moins essentiellement éliminés, étant mise en œuvre à l'aide d'une colonne de déméthanisation (DM).

10. Procédé (10) selon la revendication 7, selon lequel, dans la ou les secondes étapes de préséparation (V2) et/ou dans la ou les étapes de séparation du propane (S1), une colonne de déméthanisation et/ou une colonne de déséthanisation et/ou une colonne de dépropanisation sont utilisées, le troisième mélange de composants étant injecté au moins partiellement à l'état liquide dans la zone inférieure de la colonne de déméthanisation et/ou de la colonne de déséthanisation et/ou de la colonne de dépropanisation.

11. Procédé (10) selon l'une quelconque des revendications 7 à 10, selon lequel, dans la ou les premières étapes de préséparation (V1) du premier mélange de composants, sa teneur en hydrogène est appauvrie à une valeur de 0 à 10 % en mole, en particulier de 0,1 à 5 % en mole, par exemple de 0,2 à 2 % en mole.

12. Procédé (10) selon l'une quelconque des revendications précédentes, selon lequel la ou les premières étapes de préséparation, à laquelle ou auxquelles le premier mélange de composants est soumis, comprennent une augmentation de pression jusqu'à une pression absolue de 3 à 40 bars, en particulier de 10 à 30 bars, par exemple de 12 à 30 bars, une condensation au moins partielle des composants à point d'ébullition supérieur à celui de l'hydrogène étant effectuée en particulier dans la ou les premières étapes de préséparation après l'augmentation de pression.

13. Installation de production de propylène comprenant:
- une première unité de réacteur, laquelle est fournie et conçue pour mettre en œuvre un procédé (1) de déshydrogénation du propane avec obtention d'un premier mélange de composants (A), lequel contient au moins de l'hydrogène, de l'éthane, de l'éthylène, du propane et du propylène,
- une seconde unité de réacteur, laquelle est fournie et conçue pour mettre en œuvre un procédé de craquage à la vapeur (2) avec obtention d'un deuxième mélange de composants (B), lequel contient au moins de l'hydrogène, du méthane, de l'éthane, de l'éthylène, du propane et du propylène,
- une unité de séparation, laquelle est fournie et conçue pour former un produit de séparation (P2) contenant essentiellement du propane, à l'aide d'au moins une partie du propylène du premier mélange de composants (A) et au moyen d'une ou de plusieurs étapes de séparation du propane (S1),
- des moyens étant fournis, lesquels sont conçus pour alimenter la ou les étapes de séparation du propane (S1) en au moins une partie du premier mélange de composants (A),
**caractérisée par**
- des moyens, lesquels sont fournis et conçus pour recycler au moins partiellement le produit de séparation (P2) contenant essentiellement du propane dans le procédé de craquage à la vapeur (2).

14. Procédé de modernisation d'une installation, laquelle est conçue pour mettre en œuvre un procédé de craquage à la vapeur à l'aide d'un nombre de composants d'installation, l'installation étant alimentée, avant la modernisation, en un mélange de départ contenant des hydrocarbures présentant une première composition, **caractérisé en ce que** la modernisation comprend l'alimentation de l'installation en, au lieu du mélange de départ contenant des hydrocarbures présentant la première composition, un mélange de départ contenant des hydrocarbures présentant une seconde composition différente et l'utilisation d'un ou de plusieurs des composants d'installation pour, au lieu du procédé de craquage à la vapeur, un procédé de déshydrogénation du propane, un procédé (10) selon l'une quelconque des revendications 1 à 12 étant mis en œuvre après la modernisation ou une installation selon la revendication 13 étant fournie au moyen de la modernisation.
